Europäisches Patentamt

European Patent Office

Office européen des brevets

(11) Publication number: **0 051 045**
**A1**

## (12) EUROPEAN PATENT APPLICATION

(21) Application number: **81830184.8**

(22) Date of filing: **05.10.81**

(51) Int. Cl.³: **A 61 M 17/00**

(30) Priority: **20.10.80 IT 8496580**

(43) Date of publication of application:
**05.05.82 Bulletin 82/18**

(84) Designated Contracting States:
**AT BE CH DE FR GB LI LU NL SE**

(71) Applicant: **Benetti, Alfredo**
**Via Cà di Cozzi, 8**
**Verona(IT)**

(72) Inventor: **Benetti, Alfredo**
**Via Cà di Cozzi, 8**
**Verona(IT)**

(74) Representative: **Zorzoli, Franco**
**c/o BUGNION S.p.A. Via Carlo Farini 81**
**I-20159 Milan(IT)**

(54) **A machine suitable to cause the evacuation of gases coming from an apparatus producing anesthesia by inhalation.**

(57) A machine suitable to cause the evacuation of gases emanating from an apparatus producing anaesthesia by inhalation, and which acts without significant pressure perturbations within the intake circuits connected to the patient, the machine including a variable-volume chamber, formed by the bellows (4) and its base (6), the chamber, being suited to receive gases, from the inlet door (12) and it having a passively movable surface (7) located downstream with respect to the flow of said gases from the exhaust valve of the apparatus producing anesthesia; a variable-delivery pumping apparatus (8,9,10,11) separated from said variable-volume chamber and situated downstream thereof; automatic means (62,71) suited to control and adjust the variable delivery and arranged to minimize possible pressure-extent variations existing in said variable-volume chamber; a safety device (82,83,93) disposed between the variable-volume chamber and the outside, adapted to keep pressure in the variable-volume chamber within predetermined extents.

FIG 1

## A machine suitable to cause the evacuation of gases coming from an apparatus producing anesthesia by inhalation

It is known that during anesthesia by inhalation it is given to patients a gas mixture containing the anesthetic compound in a suitable percentage. These gas mixtures are given by means of apparatus producing anesthesia by inhalation which are provided with a first section where the anesthetic mixtures are prepared and a second section where they are given, the latter being always provided with an exhaust valve arranged to disperse the gas mixtures exhaled by a patient in the external area. As these gas mixtures exhaled by a patient always contain more or less considerable quantities of the anesthetic compound and as the external area in which these gas mixtures are generally discharged is the operating theatre or the reanimation room, it follows that there is pollution of same with the subsequent pathological effect (poisoning) which concerns the medical personnel in general and particularly the surgical staff who is constantly exposed to such pollution.

The problem has not yet been solved in a completely satisfactory manner above all as regards the existing operating theatres which are often situated underground and in places which can also be connected to the outside with difficulty. In these cases above all the volatile anesthetic compounds owing to their high molecular weight stagnate in the lower parts of the rooms and their elimination is not at all easy. In fact it would be necessary to install efficacious and expensive room air-conditioning and ventilating systems having streams of great importance which inevitably would interfere with the contour microclimate of the operating theatre. In fact, the great number of air exchanges necessary in an hour and consequently the air turbulence in the room and the unavoidable noise are to be considered as a dangerous agent for the patients and the medical staff.

As regards the elimination of gases discharged from the breathing circuit connected to the patient, it is very important to point out that downstream of the exhaust valve of the apparatus producing anesthesia no pressure perturbations have to occur be they positive or negative as they could seriously interfere with the patient respiration and the normal development of anesthesia. Particularly, positive pressure perturbations (overpressures) downstream of the usual exhaust valves of the apparatus producing anesthesia, during the exhaust stage of the same valves, could completely influence the patient breathing dynamics and the patient could encounter particular difficulties in breathing owing to the additional stress of the diaphragm. Negative pressure perturbations (depressions) beyond given values can bring to the opening of the exhaust valve and cause the escape of the normal amount of gas inhaled by the patient who in this event would be unable to execute a normal breathing in.

The object of the present invention is to provide, without interfering with the normal operation of the apparatus producing anesthesia by inhalation, for the evacuation by a controlled pumping action, of the gases discharged from the breathing circuit connected to the patient avoiding the dispersion of same in the surrounding space and acting in such a manner that remarkable pressure perturbations do not occur either during the continuous discharges or during the sudden temporary ones caused for example by coughing on the part of the patient.

This evacuation, downstream of the machine of the invention, can take place directly discharging the gases outside the building

- 3 -

where they are dispersed owing to their dilution in the atmosphere or alternatively these gases can suitably be treated in filtering systems which allow the recovering of the anesthetic compound existing therein.

A particular object of the present invention is to ensure that the pressure value downstream of the exhaust valve of the apparatus producing anesthesia to which the valve is connected does not diverge from the value of the ambient pressure more than 1 + 2 mm $H_2O$ when the gas is continuously flowing up to more than 50 N-litres/minute; on the contrary in the case of a quick discharge corresponding to the issue of an amount of exhaled gases equal to 0,6 N-litres in 0,25 seconds (this being a more important transient than the patient coughing) the perturbation in the pressure values is ranging between + 3 and + 7 mm $H_2O$. These values do not represent considerable pressure perturbations from a physiological point of view, as they have the same importance as the losses of pressure of the normal corrugated tubes suitable to carry out facial or intratracheal connections between a patient and an anesthetic apparatus.

This and other objects are attained according to the present invention by a machine suitable to cause the evacuation of gases coming from an apparatus producing anesthesia by inhalation connected to it, characterized in that it comprises: at least a variable volume chamber having a passively movable surface located downstream, with respect to the flow of said gases, of the exhaust valve of said apparatus producing anesthesia, said variable volume chamber being arranged to receive said gases coming from the above apparatus producing anesthesia; a variable delivery pumping apparatus separated from said variable volume chamber and situated downstream, with respect to the flow of said gases, of said variable volume chamber;

- 4 -

automatic means suitable to control and adjust said variable delivery determined by said pumping apparatus, said means being arranged to minimize possible value variations in pressure existing in said variable volume chamber; a safety device to keep pressure in said variable volume chamber within predetermined values.

Advantageously said variable volume chamber includes a bellows provid ed with a movable wall comprising a rigid diaphragm which can move along the longitudinal axis of said bellows; balancing means being provided in order to reduce the resultant of gravitational resilient and inertial forces (pressure stresses excepted) acting on said movable wall . The provision of this balancing means is due to the fact that the difference in pressure between the interior and the exterior of the variable volume chamber depends upon said resultant of forces.

Advantageously and according to a further feature of the present invention, this pumping apparatus at least includes a diaphragm pump ing element which is driven by a kinematic linkage comprising a connecting rod and a crank; said connecting rod being fixed at one end thereof integrally to the centerline of a rigid cap mounted in the middle of said diaphragm and at the other end thereof to the eccentric pin of said crank; said crank being fitted to a driving shaft rotated by an electric motor.

Advantageously and according to a further feature of the invention said automatic means for the controlling and adjusting of the variable delivery includes: a first (mechanical) control and adjustment system comprising a lever kinematic coupling controlled by the axial displacement of the rigid diaphragm of said bellows and acting on a by-pass valve placed downstream, with respect to the gas flow, of said pumping apparatus and communicating both with the variable volume chamber and the exterior; said first (mechanical) system being arranged to cause, mechanically acting on said by-pass valve, a partial recycle of the gases coming from the pumping apparatus, in the variable volume chamber; a second (electric al) control and regulation system, achieved by a variable inductance position transducer, of the rigid diaphragm, including a rigid rod one end of which is integral to said rigid diaphragm while the other end of which is partially introduced and slidable within a fixed slide, said second (electrical) system being arranged to cause, by an electrical signal sent to the electric motor of the pumping apparatus, the speed change of the electric motor itself.

Further features and advantages of the invention will become more evident from the detailed description of a preferred embodiment of a machine suitable to cause the evacuation of gases, given hereinafter by way of example only and being therefore intended not to limit the scope of the invention, with reference to the accompanying drawings, in which:

- Fig. 1 is a partly cut away axonometric view of the machine of the present invention;
- Fig. 2 is a back view of the lower portion of the machine (without showing the bellows);
- Fig. 3 is a side view of the lower portion of the machine (without showing the bellows);
- Fig. 4 is a bottom plan view of the machine as shown in Fig. 3;
- Fig. 5 is a top plan view of the machine (without showing the bellows);
- Fig. 6 is a part sectional view of the bellows and the members connected therewith, on the line VI-VI of Fig. 5;
- Fig. 7 is a top plan view of the machine central body;
- Fig. 8 is a bottom plan view of the machine central body;

- Fig. 9 is a sectional view of the central body on the line IX-IX of Figs. 7 and 8;

- Fig. 10 is a sectional view of the central body on the line X-X of Fig. 8;

- Fig. 11 is a sectional view of the central body on the line XI-XI of Fig. 8;

- Fig. 12 is a sectional view of the machine on the line XII-XII of Fig. 4;

- Fig. 13 is a part sectional view of the intake and delivery valves of a pumping element, on the line XIII-XIII of Fig. 4;

- Fig. 14 is a sectional view of the by-pass valve seen in Fig. 6, on the line XIV-XIV of Figs. 5 and 7;

- Fig. 15 is an offset section of safety valves on the line XV-XV of Figs. 5 and 7;

- Fig. 16 is a block diagram showing the gas path in the machine and including the automatic control and adjustment means of the variable delivery.

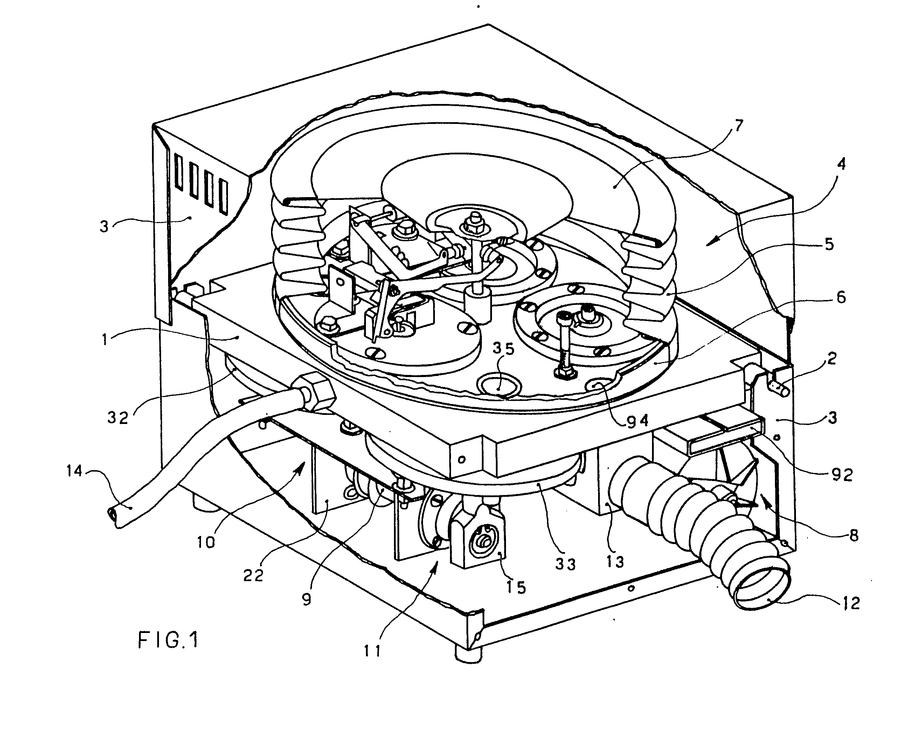

With reference to the drawings, and particularly to Fig. 1, it is indicated at 1 a plate-shaped central body provided with threaded cylindrical supports 2 carried by a crankcase 3 constituting a support and a protection for the machine inner members. At 4 it is indicated a bellows having a corrugated cylindrical surface 5 made of thin soft rubber, located at the top of the central body 1 and secured to a cylindrical fixed base 6 forming part of the central body 1. At its upper part the bellows 4 is closed by a rigid diaphragm 7 suitable to displace axially parallelly to itself in order to follow the volume changes occurring during the expanding and contracting of bellows 4. Underneath the central body 1 is located an electric motor 8 which causes the driving shaft 9 of a pumping apparatus to rotate, the latter including a first diaphragm pumping element 10 and a second diaphragm pumping element 11 acting in phase opposition. With reference to Fig. 1 it is indicated at 12 an inlet pipe allowing gases coming from the exhaust valve of an apparatus producing anesthesia to get into the machine. This inlet pipe 12 is connected to a pipe fitting block 13 made of synthetic material, fastened in the vicinity of the side edge of the central body 1 lower surface. At 14 it is indicated an outlet pipe of gases from the machine, directly connected to a side surface of the central body 1. Always referring to Fig. 1 only, it is to be noted, in a first diagrammatic view, that the path of gases through the machine is as follows: gases coming from the exhaust valve of an apparatus producing anesthesia reach, through the inlet pipe 12 the pipe fitting block 13 and hence the central body 1 which is provided, as will be described, with many gas flow passages as well as with many housings fitted for the various machine members; from said central body gases reach the variable volume chamber comprising the bellows 4 which, due to gas inlet, expands. From bellows 4 gases are pumped, through the central body 1, by means of the pair of pumping elements 10 and 11 and then ejected through the outlet pipe 14.

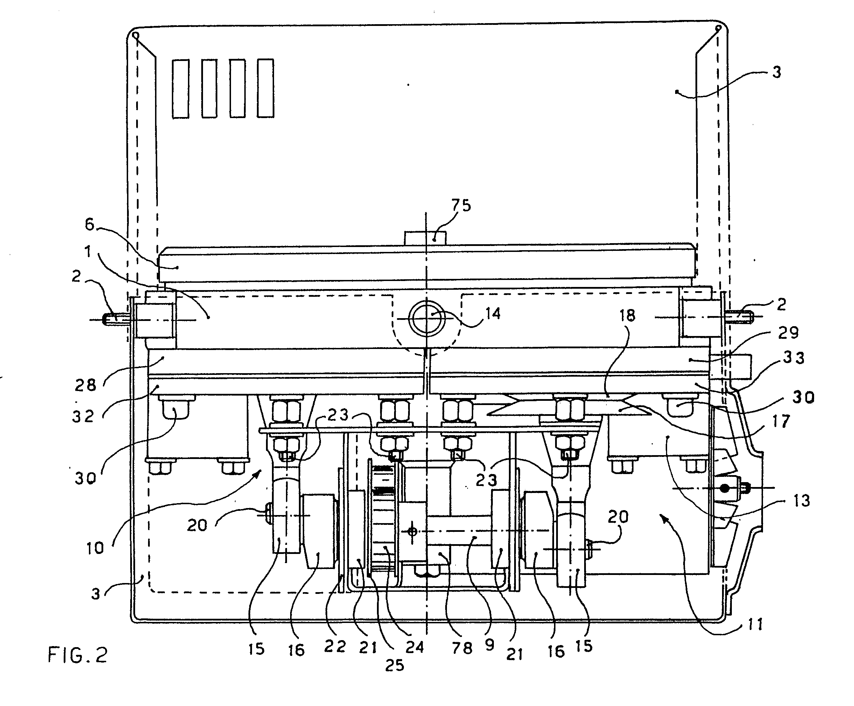

Referring to Figs. 2, 3, 4 and 12, each of the two pumping elements is driven by a kinematic coupling comprising a connecting rod 15 and a crank 16. Each connecting rod 15 is fixed at its upper end integrally to the centerline of a rigid cap 17 mounted in the middle of a diaphragm 18 and at its lower end, by means of a bearing 19, to an eccentric pin 20 of the corresponding crank 16. Each crank 16 is fitted to the shaft 9 mounted on bearings 21 fixed to a support structure 22 which in turn is secured to the central body 1 by means of a stud bolt 23. Shaft 9 is rotated by the electric motor 8 disposed parallelly to it, which transmits motion by means of a toothed belt 24 mounted on gear wheels 25. Cranks 16 act on connecting rods 15 in phase opposition (180°).

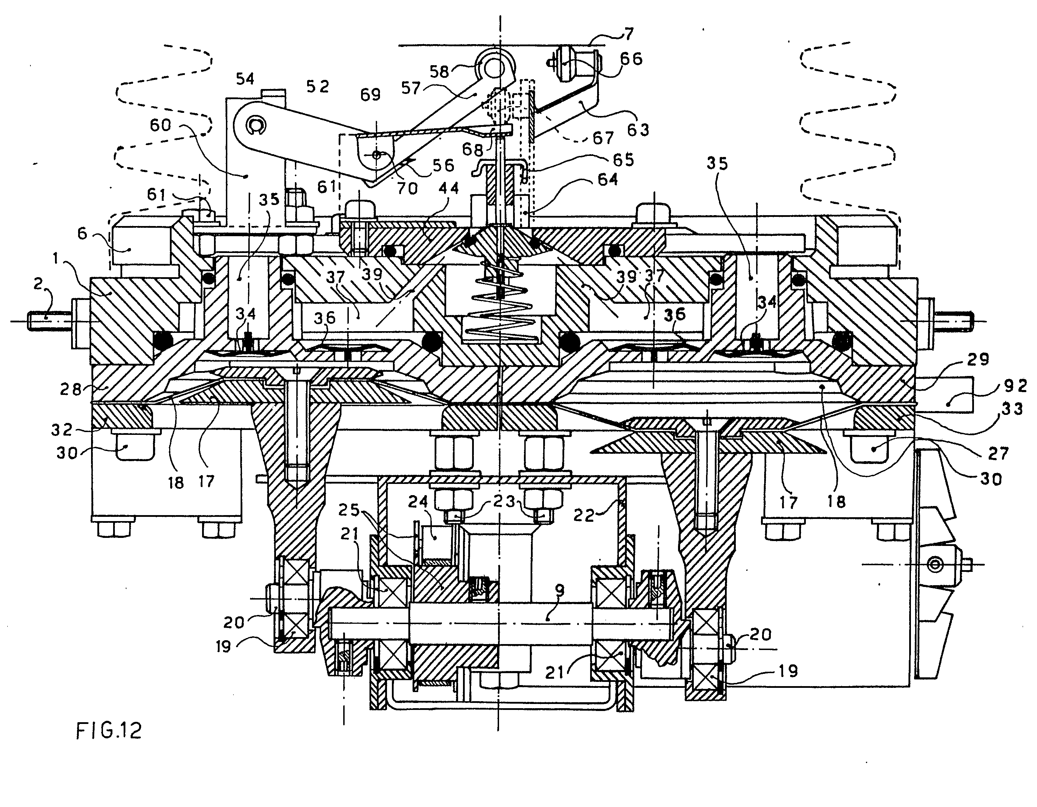

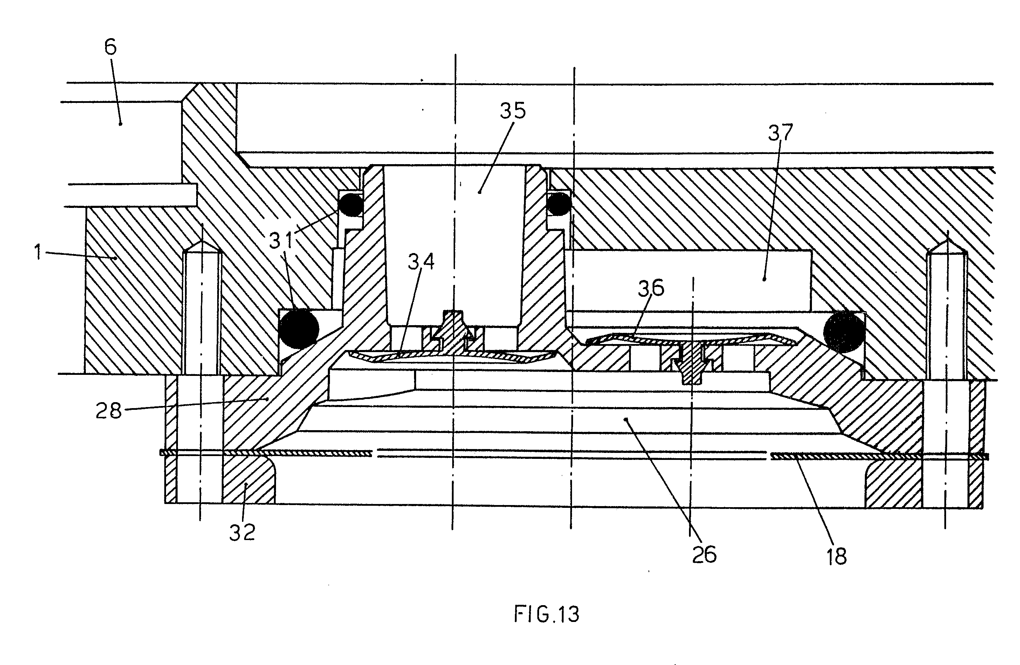

Referring to Figs. 12 and 13, it is indicated at 26 a first pumping chamber concerning the first pumping element 10 and at 27 a second pumping chamber concerning the second pumping element 11. Each pumping chamber 26 and 27, as can be seen in Figs. 12 and 13, is formed respectively in first 28 and second heads 29 rigidly fixed to the central body 1 by means of screws 30 and stud bolts 23. Pressure tight seals (O-Ring) 31 are provided which are interposed between each head 28 and 29 respectively and the central body 1. Each diaphragm 18 is peripherally sandwiched between the corresponding head 28 or 29 and a corresponding flange 32 or 33 placed below.

Always referring to Figs. 12 and 13, it is indicated at 34 each intake valve which registers with the corresponding intake opening 35 of each pumping element 10 or 11 (in particular, Fig. 13 relates to the first pumping element 10). At 36 it is indicated each delivery valve which registers with a corresponding delivery chamber 37, each being formed between the central body 1 and the heads 28 or 29. Each one of the two delivery chambers 37 communicates, downstream, with a by-pass valve 38 by means of an inlet pipe 39. This by-pass valve 38 enables one part of the pumped delivery to be discharged in bellows 4 again. In this way depressions in bellows 4 are restricted to physiologically non important values.

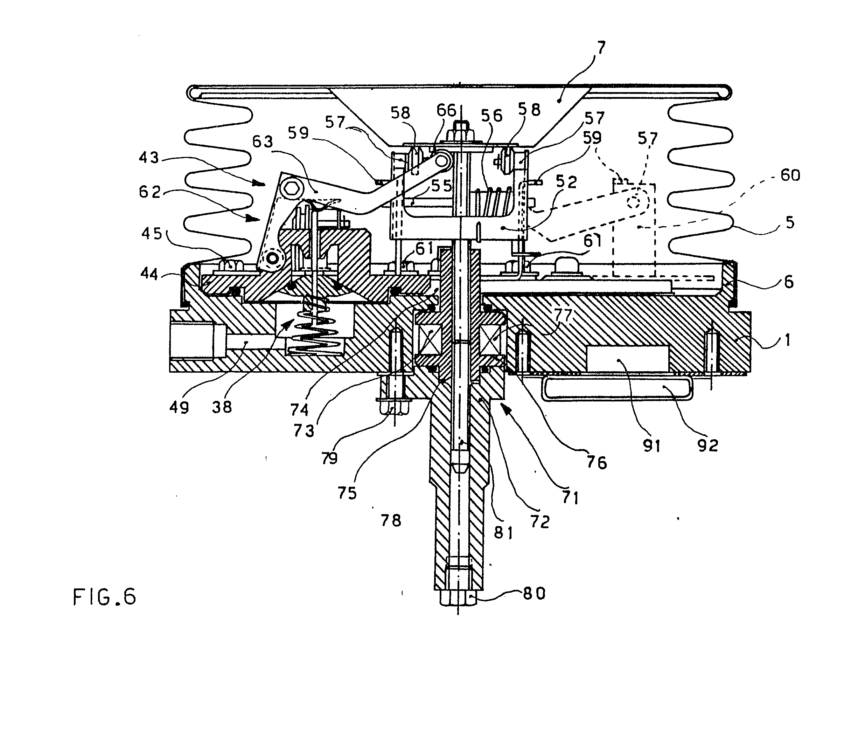

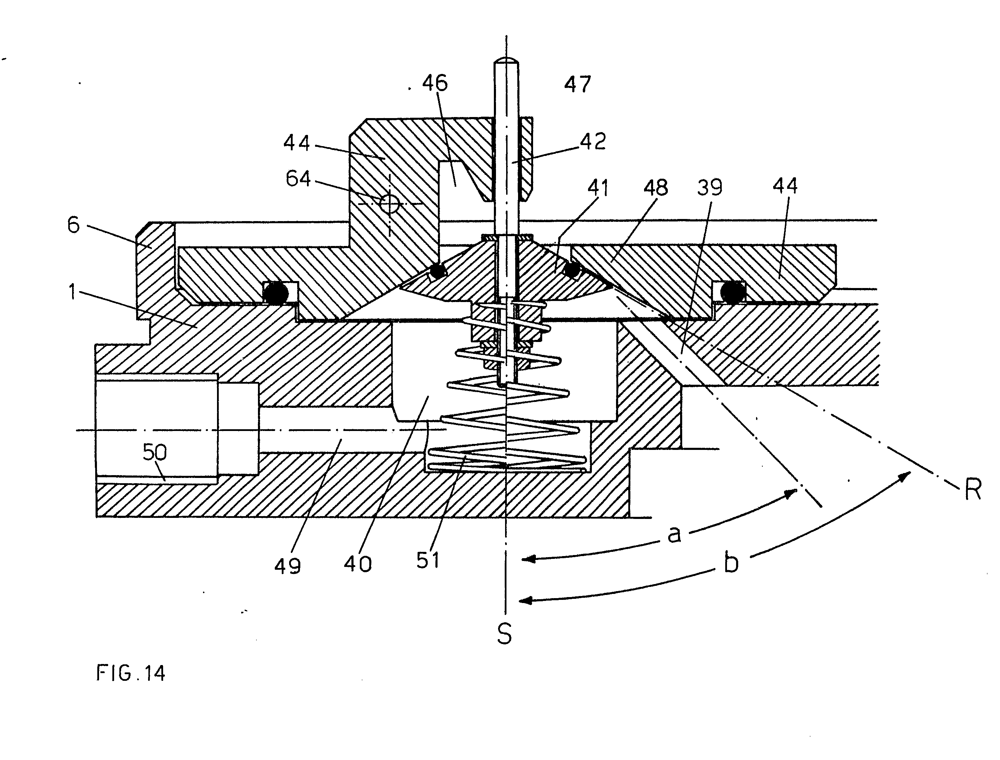

Referring to Figs. 6 and 14 it is to be noted that the by-pass valve 38 placed in a recess 40 of the central body 1 symmetrically with respect to the pumping elements 10 and 11, comprises a cone-shaped valve head 41 which tapers upwardly and is provided with a stem 42 integral to it and operated by a lever mechanism placed above with respect to the by-pass valve 38 and inside the bellows 4. A cover 44 secured to the central body 1 by means of screws 45, closes the recess 40 of the by-pass valve 38. As shown in Figs. 6 and 14, in said cover 44 is formed a first outlet pipe 46 which allows the communication between the by-pass valve 38 and the bellows 4. In the central portion of cover 46 is a through hole 47 in which the stem 42 of the valve head 41 is introduced. The by-pass valve 38 is provided with a fixed seat 48 having a substantially cone-shaped surface formed in the inner upper portion of cover 44. This surface tapers in the same way as the valve head 41. The by-pass valve 38 is connected to the exterior of the machine by a second horizontal cylindrical outlet pipe 49 formed in the central body 1, this outlet pipe 49 having a larger diameter near the outer edge of the central body 1 and being provided with a thread 50 suitable to connect the outlet pipe 14 of gases from the machine.

Referring particularly to Fig. 14, it can be noted that each inlet pipe 39 is positioned with respect to the valve head 41 so that the angle (a) determined by the geometrical intersection between the axis of symmetry of the valve head 41 and the longitudinal axis (R) of the inlet pipe 39 is equal to about 45° while the tapering (half angle (b) at the apex of the cone) of the valve head 41 and hence of the fixed seat 48 is equal to about 60°. This difference of angle inclination between each inlet pipe 39 and the fixed seat 48, due to the kinetic discharge effect, induces a slight depression in the second outlet pipe 49 which stabilizes the machine operation and avoids formation of depressions in the interior of bellows 4 when the machine is operated without any exhaust back pressure (free discharge directly in the ambience of the operating theatre) and at the same time without any amount of gas reaching the bellows 4 (as in the case of weak ventilation on the part of the patient or temporary closed circuit breathing in the apparatus producing anesthesia). The movement of the valve head 41 operated by the stem 42 is held up by a coil spring 51 placed below the valve head 41 and inside the recess 40.

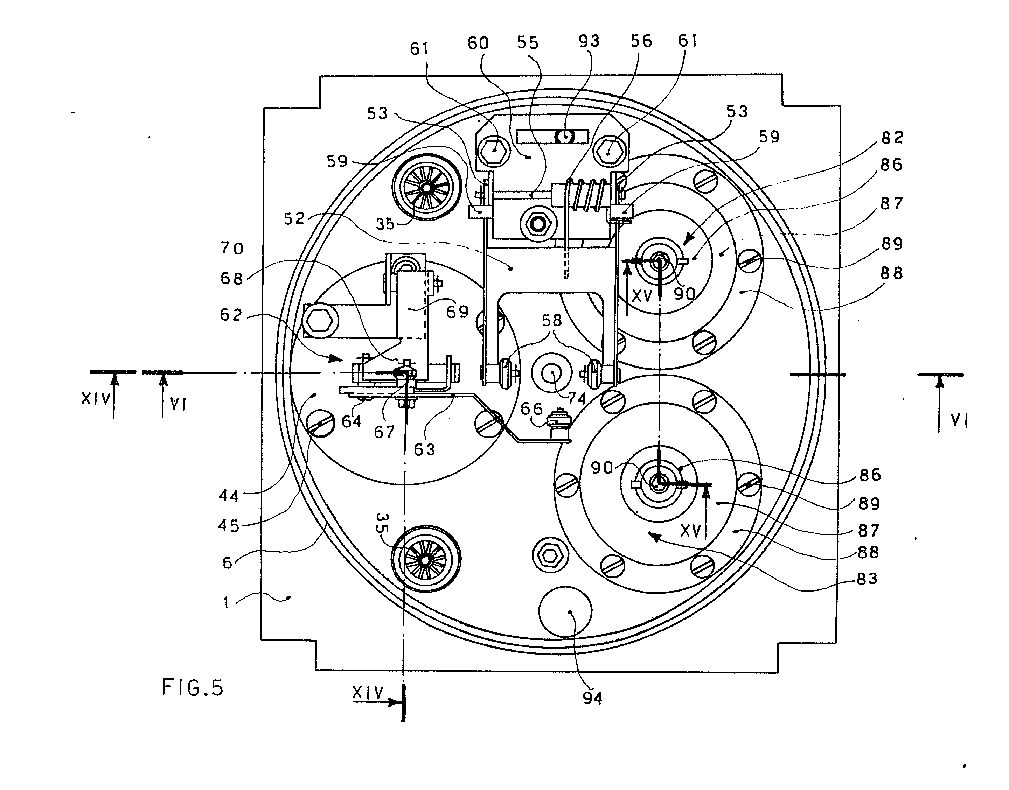

Referring to Figs. 5 and 6 it is referenced 52 an arm oscillating about fixed hinges 53 applied to one end thereof 54, including a first pivot 55 to which is fixed a first preloaded spring 56 suitable to control the rotation which causes the lifting of the second end 57 of the oscillating arm 52. The second end 57 is connected at its upper end by means of a kinematic coupling with a pair of wheels 58, to the rigid diaphragm 7 of bellows 4. Fixed stops 59 formed on a suitably shaped bracket secured to the central body 1 by means of bolts 61, determine the lower limit of stroke of diaphragm 7 (completely lowered position of bellows 4) and at the same time the upper limit of stroke for the rotation of the oscillating arm 52. Therefore this oscillating arm 52 represents the balancing means suitable to reduce the resultant of the gravitational resilient and inertial forces (pressure stresses excepted) which act on the rigid diaphragm 7, so that the pressure stresses of bellows 4 and consequently of the machine can be improved.

Always referring to Figs. 5 and 6 it is indicated at 62 a first (mechanical) control and adjustment system including a lever mechanism 43 which is controlled by the axial movement of the rigid diaphragm 7 and acts in turn on the stem 42 of the by-pass valve 38.

The lever mechanism 43 comprises a suitably shaped rigid lever 63 having one end thereof fixed in a fulcrum 64, where a second spring 65 is accommodated, situated on the upper part of cover 44 and the other end thereof kinematically coupled by means of a wheel 66 to the rigid diaphragm 7 of bellows 4. The rigid lever 63 is provided, intermediate between its ends, with a substantially cylindrical transverse sliding block 67 (Fig. 5) which is kinematically coupled by sliding to a suitably shaped curved plate which is concave on its upper part with respect to the sliding block 67. The plate 68 is provided with an arm 69 allowing the rotation thereof about a second pivot 70 placed on cover 44. The lower surface of plate 68 is kinematically coupled in turn to the upper end of the by-pass valve stem 42. Owing to its curved surface, the plate 68 is suitable to transmit the displacement of the rigid diaphragm 7 to the valve head 41 according to a predetermined law. In virtue of this artifice when the delivery pressure of the pumping elements 10 and 11 varies, the operating stress of diaphragm 7 necessary to open the by-pass valve 38 can be maintained almost constant. Consequently it helps very little in causing pressure perturbations inside the bellows 4.

Referring to Fig. 6 it is indicated at 71 a second (electrical) control and regulation system comprising a variable inductance position transducer 72 of the rigid diaphragm 7. This transducer 72 comprises a hollow rigid rod 73 one end of which is integral to the diaphragm 7 to the centre of which it is secured and the other end thereof is partly introduced and slidable in a cylindrical port 74 formed in a fixed slide 75 including two distinct elements: one upper element 76 forming the fixed part of the position transducer 72 introduced in the central body, which is provided with a coil 77 electrically connected to the electric motor 8; a second cylindrical lower element 78 projecting downwardly from the central body 1 to which it is fastened by means of screws 79.

A screw plug 80 closes the port 74 of the second element 78 at the bottom. In order to cause the generation of the electrical signal by the position transducer, on the lower portion of rod 73 is a ferromagnetic insert 81 constituting the movable part of the position transducer 72. The coil 77, of enamelled copper wire, is so shaped that it allows the necessary swings of the electrical signal in connection with changes of position of the ferromagnetic insert 81 contained in the rigid rod 73. As the rod 73 is rigidly connected to the diaphragm 7, the change of the electrical signal is attained in connection with a definite change of volume of bellows 4. The position transducer 72 has to adjust the number of revolutions of the electric motor 8, suitable to actuate the pumping elements 10 and 11, according to the detected position of the rigid diaphragm 7 of bellows 4.

When the bellows volume increases, owing to the inlet of gases, the rigid lever 63 lifts step by step against the action of the second spring 65 till the by-pass valve 38 closes, this closure being assumed to take place at an intermediate point (tipically a central point) of the displacement of diaphragm 7 and in any case before the increase in the speed of the driving motor 8 induced by the second electrical control and regulation system 71. The progressive closure of the by-pass valve 38 causes the reduction of the (variable) portion of the delivery coming from the pumping chambers 26 and 27 and recycled in the bellows 4.

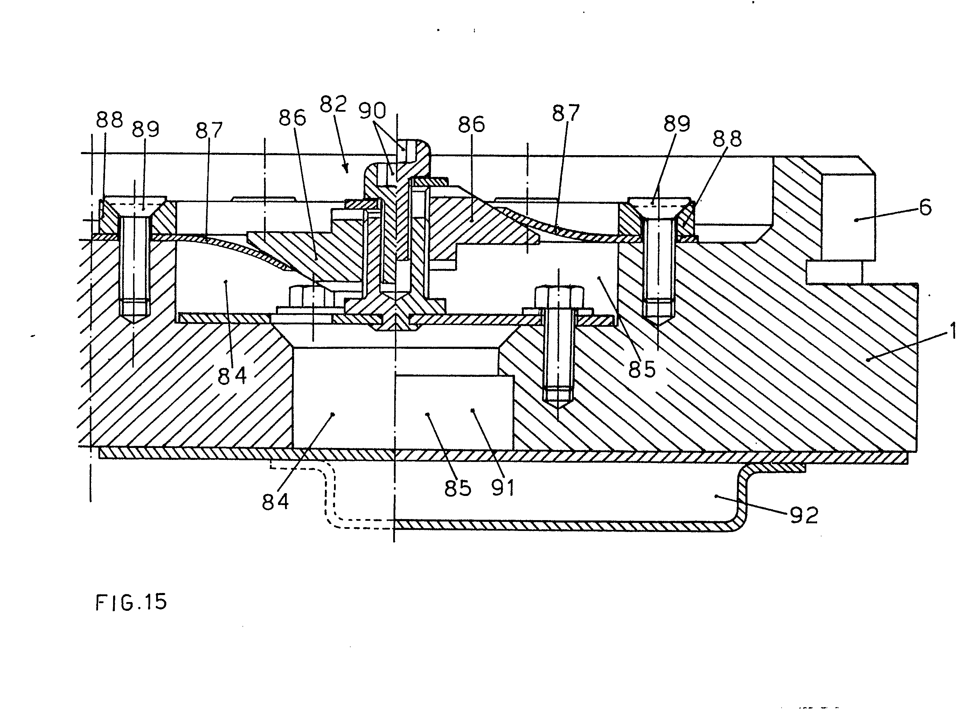

Referring to Figs. 5 and 15, it is indicated at 82 one safety valve suitable to avoid overpressures in bellows 4, respectively placed downstream of the bellows 4 and upstream of the external area, with respect to the gas flow. At 83 is indicated a second safety valve, suitable to avoid depressions in bellows 4, respectively placed upstream of the bellows 4 and downstream of the external area, with respect to the flow of the exterior air. The safety valves 82 and 83 located in the upper part of the central body 1, are disposed inside the bellows 4 symmetrically to the by-pass valve 38. The safety valves 82 and 83, which are exactly alike but placed inverted with respect to one another, are accommodated in first and second housings 84 and 85 respectively, both formed in the central body 1. Each one comprises a fixed truncated cone-shaped head 86 in line with the first and second housings 84 and 85 respectively and an elastic diaphragm 87 forming the movable member, which is essentially circular and in line with the corresponding head 86 having its outer edge fixed to the central body 1 by way of a flange 88 provided with screws 89.

The elastic diaphragm 87 which achieves the opening and closing of the flow way of the corresponding valve 82 or 83, is provided with a circular central hole coaxial to the corresponding valve head 86 and lies in a plane normal to the axis of symmetry of the valve head 86. During the closing phase of valves 82 and 83 the above mentioned hole has its circular edge in contact with the valve head 86 which is provided on its upper part with an adjustment member 90 suitable to set the axial position thereof. The displacement of each elastic diaphragm 87 is automatically determined by differences in pressure (which are predetermined according to the axial position of the valve head 86 and to the elasticity of the material forming the diaphragm 87) between upstream and downstream of each valve 82 or 83. At the lower part of the central body 1, a manifold 91 connects the safety valves 82 and 83 with a quadrangular section passage 92 fixed at the lower part of the central body 1, which projects outwardly and communicates with the area outside the machine.

As specified above, the first safety valve 82 is designed to avoid the formation of high pressures in the bellows 4, due to a possible bad working of the pumping apparatus.

In order to avoid troubles to the patient's breathing, the opening of the first valve 82 is adjustable within a field typically ranging between + 10 and + 50 mm $H_2O$ of overpressure in the bellows 4 with respect to the surrounding atmosphere.

As the calibration pressure is reached, the first valve 82 begins to discharge in the surrounding atmosphere through the manifold 91 and the passage 92. In this case the discharge appears acceptable as it can occur in the event of failure of the pumping apparatus only, with a reduction in the pumping capacity below the delivery values averagely discharged at that time from the apparatus producing anesthesia. The intervention of the first valve 82 can be preceded by an acoustic and/or visual warning generated by a pressure switch or by a suitable pressure transducer connected to the interior of bellows 4. The second safety valve 83 is designed, as previously described, to avoid the formation of depressions within the bellows 4 with respect to the atmosphere, which depressions are generated, in this case too, by the bad working of the pumping apparatus or of the by-pass valve 38. The second valve 83 can be calibrated, as to its opening, in a field typically ranging between $- 10$ and $- 50$ mm $H_2O$ with respect to the atmosphere. When a lower pressure with respect to the ambient one, of a value corresponding to the calibration value is reached in the bellows, the second valve 83 opens allowing air to enter and avoiding the depression to increase beyond the predetermined calibration value in bellows 4.

Referring to Fig. 5, it is indicated at 93 a third safety valve located in the upper portion of the central body 1 within the bellows 4 and connecting one of the delivery chambers 37, which communicate with one another, to bellows 4. The third safety valve 93 which can normally be calibrated between 0,2 + 1 Bar, is designed for avoiding overpressures caused by casual obstructions downstream of the delivery chambers 37 of the pumping elements 10 and 11, as the same could damage the pumping apparatus. As the third safety valve 93 intervenes, it discharges within the bellows 4 directly, eventually generating a whistling which constitutes the acoustic warning thereof. Should the obstruction determining the intervention of the third valve 93 last a relatively short time such that it allows the bellows 4 to simultaneously receive gases coming from the apparatus producing anesthesia, no discharges towards the surrounding atmosphere will take place. On the contrary, should the obstruction last a longer time, pressure in bellows 4 will gradually increase till the first safety valve 82 is operated and begins the exhaust towards the exterior.

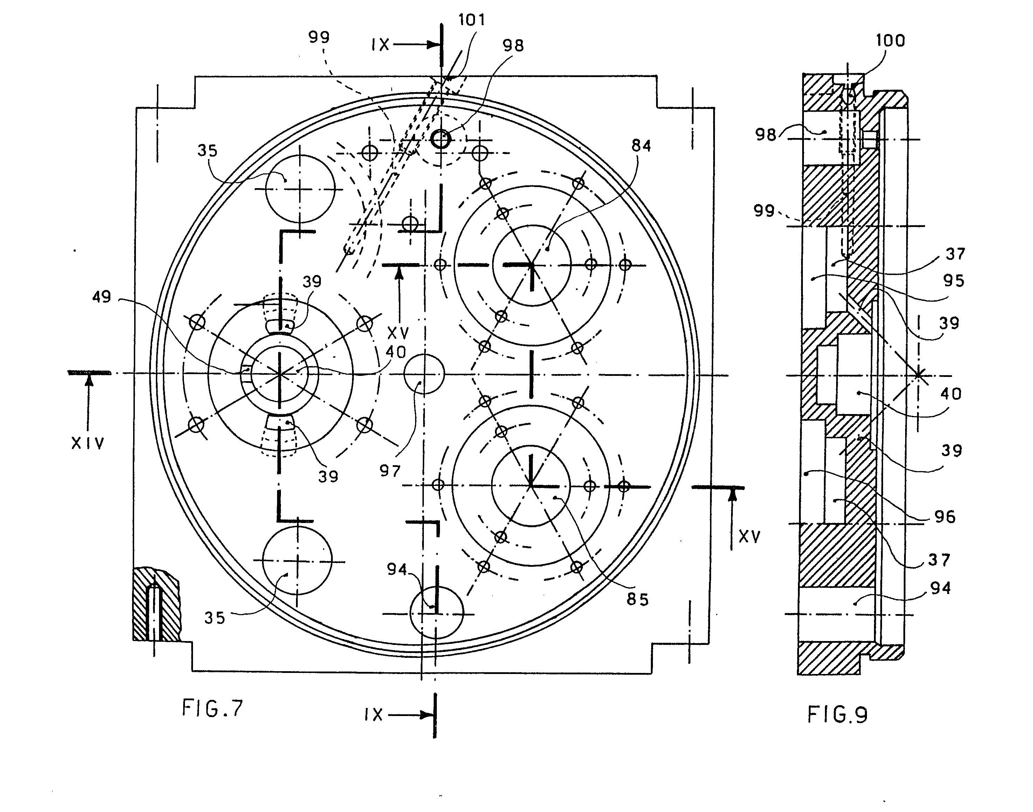

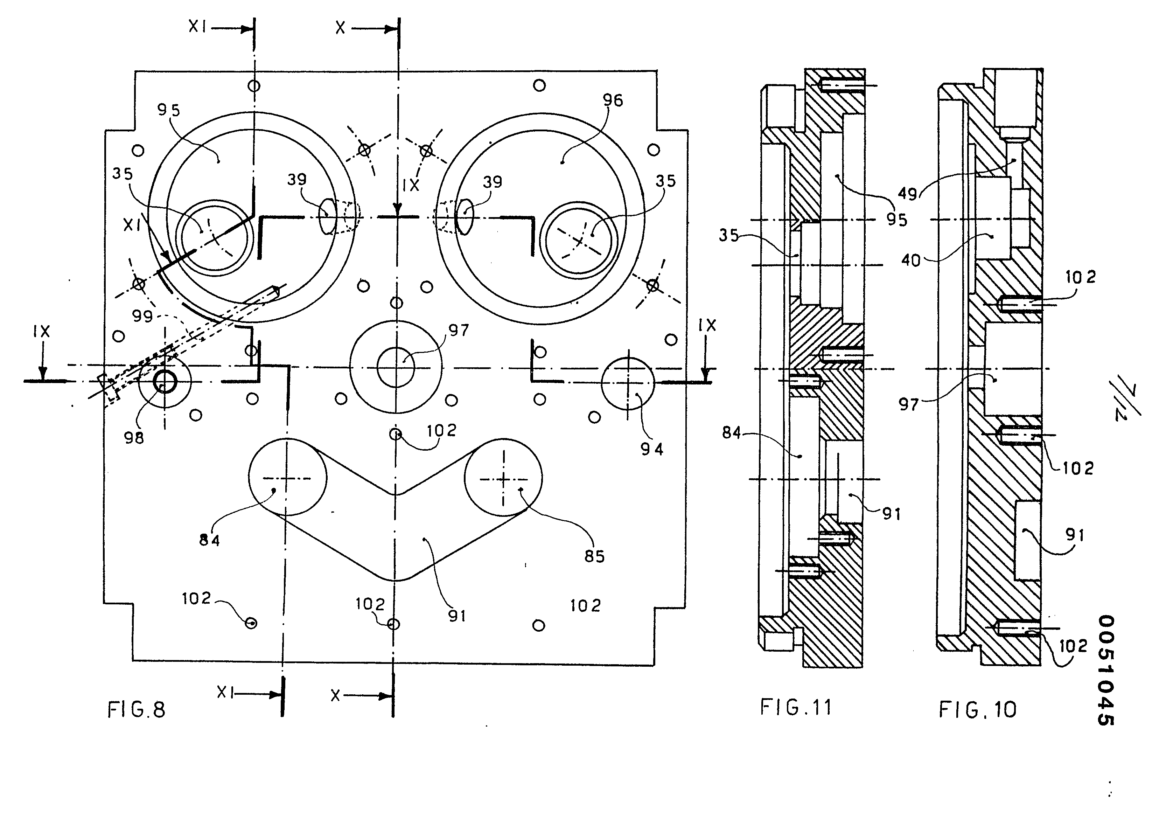

Referring particularly to Figs. 7, 8, 9, 10 and 11, in which the central body 1 is shown in detail, it is indicated at 94 a first inlet passage of gases in bellows 4, which communicates upstream with the pipe fitting block 13 (and hence with the inlet pipe 12), this passage being located peripherally in the vicinity of the cylindrical base inner edges 6. The intake openings 35 of the pumping elements 10 and 11 are also disposed in the vicinity of the cylindrical base inner edge and their corresponding longitudinal axes are placed eccentrically (as shown in Fig. 8) with respect to the corresponding longitudinal axes of the pumping chambers 26 and 27 formed in the heads 28 and 29 which are accommodated in corresponding recesses 95 and 96 of the central body 1. As is particularly shown in Fig. 9, inlet pipes 39 start from the recesses 95 and 96, these pipes being positioned according to the angle (a) in Fig. 14 of the by-pass valve 38. The valve is fitted in the recess 40 which is symmetrically positioned at the upper portion of the central body 1 with respect to the recesses 95 and 96 and eccentrically to the central vertical axis of the central body 1 (Fig. 7).

In Fig. 7 and particularly in Fig. 10 there is shown the recess 40 and the corresponding second cylindrical outlet pipe 49 as well as the thread 50 fitted for connection to the outlet pipe 14 of gases from the machine. On the opposite side with respect to the recess 40, as is shown in Fig. 7, are located the first and second seatings 84 and 85 for the first and second safety valves 82 and 83 respectively. These seatings 84 and 85, as shown in Figs. 8, 10 and 11, are connected to one another at the lower portion of the central body 1 by the V-shaped manifold 91 (Fig. 8) which is in communication with the quadrangular section passage 92.

A first hole 97 in the middle of the central body 1, as is seen in Figs. 7, 8 and 10, constitutes the housing of the position transducer first upper element 76. A second hole 98 is arranged at the opposite side with respect to the first inlet opening 94 of gases in the bellows 4 (Figs. 7, 8 and 9) in order to accommodate the third safety valve 93. The second hole 98 is in communication with one of the delivery chambers 37 by means of a horizontal drilling 99 completely interior to the central body 1 (see Fig. 7) which has a working clearance 100 on the side wall of the central body 1 near the third safety valve 93.

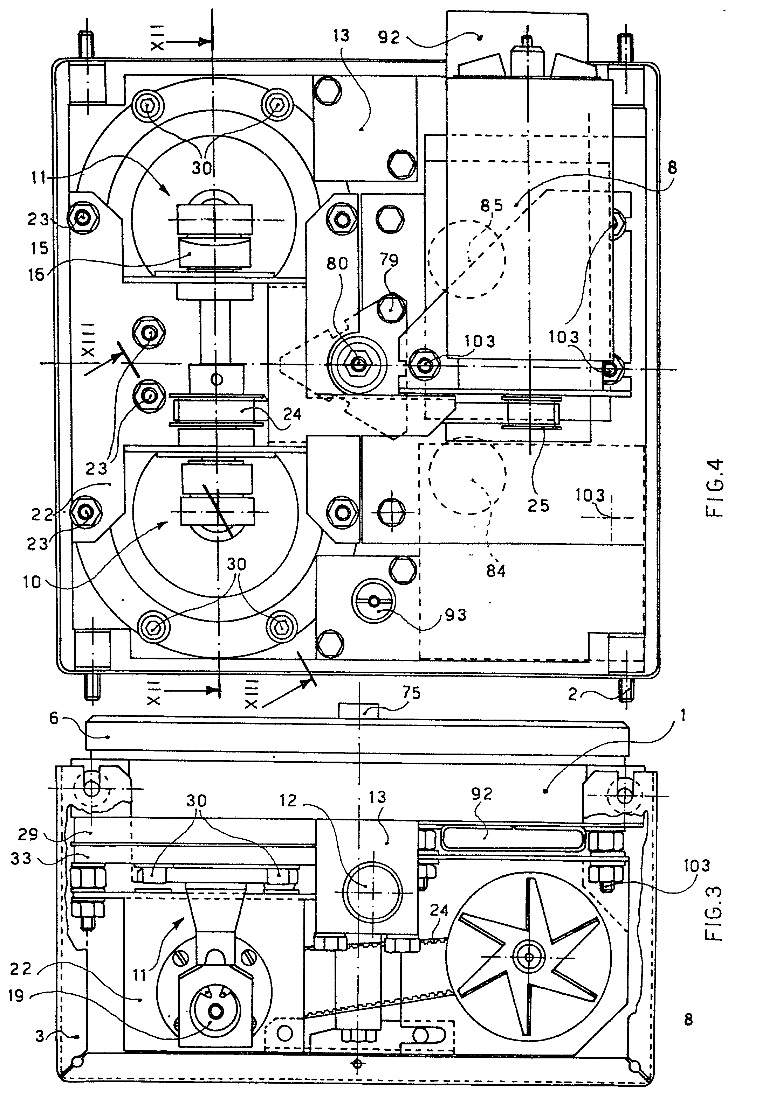

The working clearance 100 is closed on the outside by a plug 101 which avoids the escape from the machine of the possible flow coming from the delivery chamber 37, this flow being on the contrary conveyed towards the bellows 4 through the third safety valve 93. Referring to Figs. 8, 10 and 11, threaded holes housing corresponding screws 103 (Fig. 4) are referenced 102.

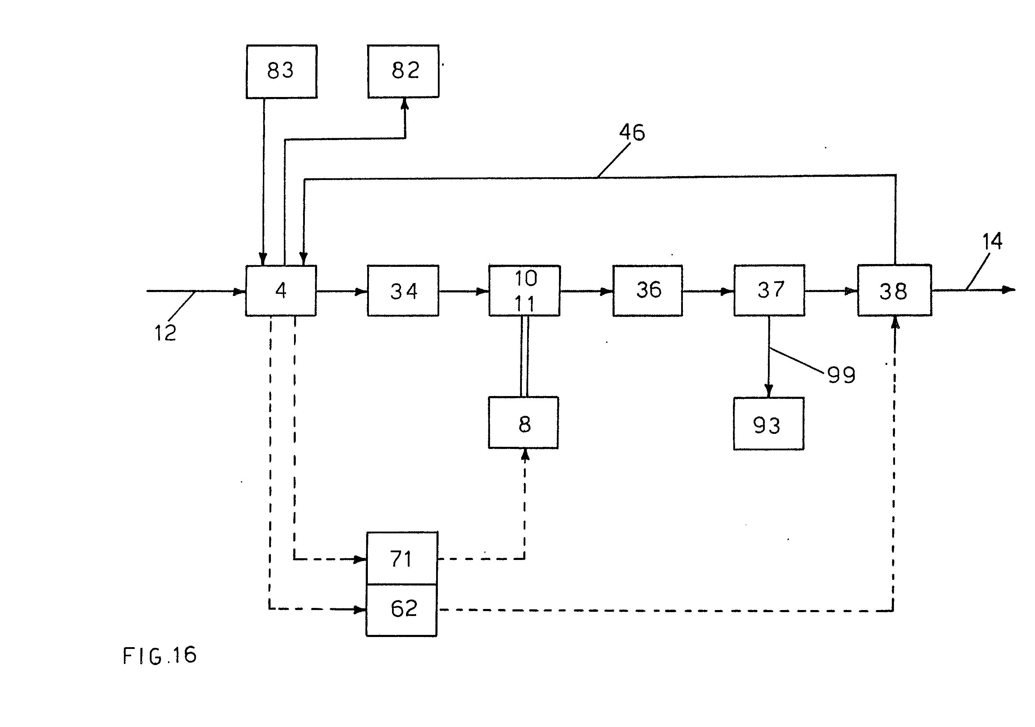

After having described the essential parts of the machine of the present invention it is now described the operation of same from which further advantages will appear, particularly with reference to the block diagram of Fig. 16.

During anesthesia gases exhaled by a patient pass to an exhaust valve of the apparatus producing anesthesia and reach, through an inlet corrugated tube 12 the bellows 4 of the machine. Therefore, bellows 4 increases its volume due only to the effect of the entering gases. Then gases contained in bellows 4, during the inlet stroke of one of the pumping elements 10 or 11, enter, through the intake valves 34, one of the pumping chambers 26 or 27 defined by the diaphragm 18. When the diaphragm 18, urged by the connecting rod 15, is compressed, gases previously sucked reach the pumping pressure, cause the opening of the delivery valves 36 and come into the delivery chambers 37. From the delivery chambers 37 gases can reach the outlet pipe 14 of the machine or they can be partly or completely recycled in bellows 4 through the first outlet pipe 46 in virtue of the gradual opening of the by-pass valve 38 which is controlled by the lever mechanism 43 responsive to the position of the rigid diaphragm 7 of bellows 4. Owing to the fact that the pumping apparatus has the same features as a positive-displacement pump, the delivery flowing out of the machine is the difference between the delivery which has flowed into the bellows 4, which is dependent upon the number of revolutions of the motor 8 and in a less degree upon the delivery pressure, and the delivery which has returned into the bellows 4 through the by-pass valve 38. It is therefore achieved a first mechanical control and adjustment system 62 of the pumping apparatus depending upon the position of bellows diaphragm 7. This system 62 acts on the net delivery coming out of the pumping apparatus independently of the control carried out by means of the speed changes of the electric motor 8 by the second electric system 71.

Both the control systems taken together, the electric one 71 which varies the revolutions of the motor 8 and the mechanical one 62 by means of the by-pass valve 38, determine the pumping features of the pumping apparatus which can be pointed out as follows.

- When the bellows 4 is in the position of its smallest volume the
motor 8 runs at the lowest speed and the by-pass valve 38 is completely open. This condition is normally achieved when the delivery
pressures are very low and there is scarcity or absence of gases
discharged by the patient.  The bellows appears compressed and
there is a slight depression inside it.

- The bellows 4 begins its displacement (the rigid diaphragm 7 begins
to lift); motor 8 still runs at the lowest speed; pumping gradually
increases while the by-pass valve 38 gradually closes owing to the
lifting of the rigid lever 63 urged by the second spring 65.

- There is a further displacement of bellows 4 (the rigid diaphragm 7
lifts up again); the motor 8 still runs at the lowest speed; the
by-pass valve 38 closes. The whole delivery of gas entering the bellows
4 is sent to the outlet pipe 14. However this delivery is still weak
owing to the low number of revolutions of the motor.

- The bellows 4 continues its displacement (the diaphragm 7 continues
to rise); the number of revolutions of the motor increases; the by-
pass valve 38 remains closed and the delivery begins to increase.

- There is a further displacement of bellows 4 (the rigid diaphragm
7 continues to rise); the motor 8 reaches its highest number of
revolutions; the by-pass valve 38 is always closed and the delivery
reaches its utmost value (given by the pumping capacity of the
pumping apparatus).

- The bellows 4 is in a position to carry out a further displacement (the rigid diaphragm 7 can still lift up); the motor 8 runs
at the highest speed; the by-pass valve 38 is always closed. The
possibility of a further displacement by the bellows 4 ensures the immediate reception of gas the outlet of which is due for example to coughing on the part of the patient, etc. Of course, this possibility is greater with respect to the position of bellows 4 in the preceding points.

The invention herein described may be embodied in other specific forms and modifications can be carried out within the scope and the spirit of the following claims.

C L A I M S

1. A machine suitable to cause the evacuation of gases coming from an apparatus producing anesthesia by inhalation connected thereto, characterized in that it comprises: at least a variable volume chamber having a passively movable surface located downstream, with respect to the flow of said gases, of the exhaust valve of said apparatus producing anesthesia, said variable volume chamber being arranged to receive said gases coming from the above apparatus producing anesthesia; a variable delivery pumping apparatus separated from said variable volume chamber and situated downstream, with respect to the flow of said gases, of said variable volume chamber; automatic means suitable to control and adjust said variable delivery determined by said pumping apparatus, said means being arranged to minimize possible value variations in pressure existing in said variable volume chamber; a safety device to keep pressure in said variable volume chamber within predetermined values.

2. A machine according to claim 1, characterized in that said variable volume chamber is provided with a movable wall comprising a rigid diaphragm (7) suitable to displace axially parallelly to itself; balancing means being provided in order to reduce the resultant of gravitational resilient and inertial forces (pressure stresses excepted) acting on said rigid diaphragm (7).

3. A variable volume chamber according to claim 2, characterized in that said balancing means comprises an arm (52) oscillating about fixed hinges (53) applied to one end thereof (54), the second end (57) of which arm being connected by means of a kinematic sliding coupling, to the rigid diaphragm (7), said oscillating arm (52) being provided with a first spring (56) suitable to apply a spring thrust force transmitted by said arm (52) to the rigid diaphragm(7).

4. A machine according to claims 1, 2 and 3, characterized in that said variable volume chamber includes a bellows (4) having a corrugated cylindrical surface (5) made of thin soft rubber.

5. A machine according to claims 1, 2 and 3, characterized in that said variable volume chamber is a chamber provided with a diaphragm.

6. A machine according to claims 1, 2 and 3, characterized in that said variable volume chamber includes a cylinder having a movable end plate.

7. A machine according to claim 1, characterized in that said pumping apparatus at least includes a diaphragm (18) pumping element (10 - 11) which is driven by a kinematic linkage comprising a connecting rod (15) and a crank (16); said connecting rod (15) being fixed at one end thereof integrally to the centerline of a rigid cap (17) mounted in the middle of said diaphragm (18) and at the other end thereof, to the eccentric pin (20) oc said crank (16); said crank (16) being fitted to a driving shaft (9) rotated by an electric motor (8).

8. A machine according to claim 1, characterized in that said pumping apparatus comprises a diaphragm pump having a plurality of pumping sections acting in series or in parallel.

9. A machine according to claim 1, characterized in that said pumping apparatus includes a reciprocating compressor.

10. A machine according to claim 1, characterized in that said pumping apparatus includes a centrifugal compressor.

11. A machine according to claim 1, characterized in that said pumping apparatus includes an axial-flow fan.

12. A machine according to claim 1, characterized in that said pumping apparatus includes a fluid ejector, means being provided to supply said fluid ejector with the necessary air delivery.

13. A machine according to claims 1 and 12, characterized in that said fluid ejector is so shaped that its operation is based on Venturi effect.

14. A machine according to claims 1 and 12, characterized in that said fluid ejector is so shaped that its operation  is based on Vortex effect.

15. A machine according to claims 1 and 12, characterized in that said fluid ejector is so shaped that its operation is based on Coanda  effect.

16. A machine according to claims 1, 2 and 7 (or following claims), characterized in that said automatic control and adjustment means includes: a first (mechanical) control and adjustment system (62) comprising a lever kinematic coupling (43) controlled by the axial displacement of the rigid diaphragm (7) and acting on a by-pass valve (38) placed downstream, with respect to the gas flow, of said pumping apparatus and communicating both with said variable volume chamber (4) and the outside, said first (mechanical) system (62) being arranged to cause, mechanically acting on said by-pass valve (38), a partial recycle of gases coming from the pumping apparatus, in said variable volume chamber (4); a second  (electric al) control and regulation system (71) achieved by a variable inductance position transducer (72), of said rigid diaphragm (7), including a rigid rod (73) one end of which is integral to said rigid diaphragm (7) while the other end of which is partially introduced and slidable within a fixed slide (75), said second (electrical) system (71) being arranged to cause, by an electrical signal sent to the electric motor (8), the speed change of the electric motor itself (8).

17. A machine according to claim 16, characterized in that said by-pass valve (38) comprises: a cone-shaped valve head (41), which tapers upwardly and is provided with a stem (41) integral thereto and operated by a lever mechanism (43) of said first mechanical control system (62); a fixed seat (48) having a substantially cone-shaped surface which tapers in the same way (b) as the valve head (41); at least an inlet pipe (39) connected to said pumping apparatus; at least one outlet pipe (46) connected to said variable volume chamber (4); at least a second outlet pipe (49) connected to the exterior.

18. A valve according to claim 17, characterized in that said inlet pipe (39) is positioned with respect to the valve head (41) so that the angle (a) determined by the geometrical intersection between the axis of symmetry (S) of the valve head (41) and the longitudinal axis (R) of the inlet pipe (39) is smaller than the tapering (b) (half angle at the apex of the cone) of the valve head (41).

19. A valve according to claim 18, characterized in that said angle (a) is equal to about 45° and said tapering (b) is equal to about 60°.

20. A machine according to claims 16 and 17, characterized in that said lever mechanism (43) comprises a suitably shaped rigid lever (63) having one end thereof fixed in a fulcrum (64), the other end thereof being kinematically coupled to the rigid diaphragm (7);
the rigid lever (63) being provided, intermediate between its ends,
with a transverse sliding block (67) which is kinematically coupled,
by sliding, to a suitably shaped curved plate (68) which is concave
with respect to the sliding block (67); said plate (68) rotating
freely about a fixed pivot (70); said plate (68) being also kinema-
tically coupled to the upper end of stem (42) of the by-pass valve
(38); the curved surface of plate (68) being suitable to transmit
the displacement of the rigid diaphragm (7) to the valve head (41)
of said by-pass valve (38), according to a predetermined law.

21. A machine according to claim 1, characterized in that said
safety device comprises: at least one safety valve (82) respectively placed downstream of said variable volume chamber (4) and
upstream of the external area, with respect to the gas flow, said
first safety valve (82) being suitable to avoid overpressures in
said variable volume chamber (4); at least a second safety valve
(83) respectively placed upstream of said variable volume chamber
(4) and downstream of the external area, with respect to the
flow of the exterior air, said second safety valve (83) being
suitable to avoid depressions in said variable volume chamber (4).

22. A safety device according to claim 21, characterized in that
each one of said first and second safety valves (82 - 83) includes:
a fixed truncated cone-shaped head (86); an essentially circular
elastic diaphragm (87) in line with said valve head (86) and
with its own fixed edge, forming the movable member of said valves
(82 - 83), suitable to achieve the opening and closing of the flow
way, said elastic diaphragm (87) being provided with a circular
central hole coaxial to said fixed valve head (86) and lying in a
plane normal to the axis of symmetry of the valve head (86), the
above mentioned hole of the elastic diaphragm (87) having its

- 25 -

circular edge in contact with the valve head (86) during the closing of said safety valves (82 - 83), the displacement of said elastic diaphragm (87) being aotomatically determined by differences in pressure between upstream and downstream of said safety valves (82 - 83); an adjustment member ( 90) suitable to set the axial position of said valve head (86).

23. A machine substantially as hereinbefore described with reference to the accompanying drawings.

FIG.1

FIG.2

FIG.3

FIG.4

2/5

0051045

FIG.5

0051045

FIG.6

FIG.7

FIG.9

0051045

7/12

0051045

FIG.10

FIG.11

FIG.8

FIG.12

FIG.13

FIG.14

FIG.15

0051045

0051045

12/12

FIG.16

European Patent
Office

**EUROPEAN SEARCH REPORT**

Application number

EP 81 83 0184

| | **DOCUMENTS CONSIDERED TO BE RELEVANT** | | **CLASSIFICATION OF THE APPLICATION (Int. Cl. 3)** |
|---|---|---|---|
| Category | Citation of document with indication, where appropriate, of relevant passages | Relevant to claim | A 61 M 17/00 |
| X | <u>US - A - 4 176 666</u> (HOVEY) <br><br> * abstract; figures 1,2 and 4 * <br> --- | 1,2,4 5,17 | |
| X | <u>FR - E - 86 888</u> (ENGSTRÖM) <br><br> * page 2, column 1, paragraph 8 to page 3, column 1, paragraph 1; figures 1 and 2 * <br> --- | 1,5,9 12,13 | |
| X | <u>WO - A - 80/01044</u> (FISHER) <br><br> * page 5, line 26 to page 6, line 34; figures 3-5 * <br> --- | 1,5 | **TECHNICAL FIELDS SEARCHED (Int.Cl. 3)** <br> A 61 M |
| | <u>US - A - 4 004 585</u> (BOEHRINGER) <br><br> * column 2, line 39 to column 3, line 38; figures 1 and 2 * <br><br>---------- | 1,5, 11 | |
| | | | **CATEGORY OF CITED DOCUMENTS** <br><br> X: particularly relevant if taken alone <br> Y: particularly relevant if combined with another document of the same category <br> A: technological background <br> O: non-written disclosure <br> P: intermediate document <br> T: theory or principle underlying the invention <br> E: earlier patent document, but published on, or after the filing date <br> D: document cited in the application <br> L: document cited for other reasons <br><br> &: member of the same patent family, corresponding document |

| The present search report has been drawn up for all claims | | |
|---|---|---|
| Place of search The Hague | Date of completion of the search 18.01.1982 | Examiner GERMANO |

EPO Form 1503.1  06.78